Europäisches Patentamt

European Patent Office

Office européen des brevets

(11) Publication number: **0 475 505 A1**

# EUROPEAN PATENT APPLICATION

(21) Application number: **91202231.6**

(22) Date of filing: **03.09.91**

(51) Int. Cl.5: **A61L 2/22, A61L 9/14**

(30) Priority: **14.09.90 NL 9002025**

(43) Date of publication of application:
**18.03.92 Bulletin 92/12**

(84) Designated Contracting States:
**AT BE CH DE DK ES FR GB GR IT LI LU NL SE**

(71) Applicant: **DUPHAR INTERNATIONAL RESEARCH B.V**
**C.J. van Houtenlaan 36**
**NL-1381 CP Weesp(NL)**

(72) Inventor: **Pollmann, Thomas B.H.M.**
**c/o OCTROOIBUREAU ZOAN B.V., P.O.Box 140**
**NL-1380 AC Weesp(NL)**
Inventor: **Kottrik, Alexander M.G.**
**c/o OCTROOIBUREAU ZOAN B.V., P.O.Box 140**
**NL-1380 AC Weesp(NL)**

(74) Representative: **Swaters, Pieter D. et al**
**OCTROOIBUREAU ZOAN B.V. P.O. Box 140**
**NL-1380 AC Weesp(NL)**

(54) Method of disinfecting the interior of an isolator and device suitable therefor.

(57) The invention relates to a method of disinfecting the interior of an isolator comprising a laminar air flow system, by nebulising a liquid disinfectant in an air flow and then causing the said disinfecting aerial mixture to circulate laminarly through the isolator via a highly efficient particle filter.

The invention also relates to a device which is suitable for carrying out the said method.

FIG. 1

The invention relates to a method of disinfecting the interior of an isolator comprising a laminar air flow system.

Isolators, or isolator cabinets, are increasingly used in the pharmaceutical industry. Pharmaceutical products which cannot or cannot readily be sterilised at the end of the production process or in the ultimate package, must be produced and dispensed or packaged in a sterile room. Up till now this has usually been done in a so-called clean room, a sterile room. The operating personnel, dressed in sterile clothing, gloves, face-protectors, etc., is present in person in such a clean room. It will therefore be obvious that entering the clean room requires a lot of preparation by the said personnel; the total time for changing clothes for each person is approximately 1 hour per working day. Acquiring, mending and cleaning sterile clothing is expensive. Such a clean room is used to protect the materials (products) in particular against contamination by the operating personnel; this protection, however, depends on the hygiene and skill of the said personnel. Moreover, the operating personnel is very insufficiently protected against poisonous or detrimental chemical substances during the handling of said substances. Upon disinfecting the clean room, a large space is to be treated, which is time-consuming and requires much disinfectant. Of course, a clean room occupies much space (great investments), because it must be possible for the operating personnel to work in it.

The above-mentioned disadvantages can be mitigated by using an isolator or isolating cabinet for producing, dispensing, etc. under aseptic conditions, instead of a clean room. An isolator presents the possibility to perform aseptic treatments/processes while the operator, i.e. the person who performs the operations, is present outside the sterile room. Gloves provided in the wall of the isolator enable the operator to perform various operations in the isolator, for example, handling sterile material, operating instruments, and the like. In larger isolating cabinets a so-called halfsuit is often present, in which the upper part of the operator's body stands in the otherwise completely closed isolator and in this manner he or she has a larger "reach" inside the cabinet. In addition to considerable economic advantages, important quality advantages are also associated with operating in an isolator compared with a clean room. In an isolator the personal hygiene of the operator is not of importance and the possibility of contamination of the materials (products) which are treated sterile is reduced to substantially zero.

In order to keep the isolator sterile during performing operations therein, the cabinet usually comprises a system to maintain an excess pressure (positive pressure) in the cabinet and a filtering system for admitted air. The admitted air may be passed through the cabinet turbulently or laminarly. However, before use the isolator must first be disinfected. This is usually done by rinsing the interior of the cabinet with a disinfectant, usually in vapour form, and then removing the remainder of the said disinfectant by rinsing with air. In practice the said disinfecting process is carried out by circulating the disinfectant in vapour form through the isolator until a sufficient reduction of the number of micro-organisms has been reached.

The disinfection of the isolator depends on the disinfectant used. A much used disinfectant is a diluted, stabilised peracetic acid solution which is evaporated outside the isolator and is then introduced into and passed through the cabinet in vapour form; after circulation the disinfectant is returned to the evaporator. After this disinfection process the disinfectant must be completely removed from the isolator by rinsing with air. In the isolators now available the overall process time of disinfecting and rinsing is approximately 15 hours. Such a period of time is usually also prescribed by the manufacturers of isolating cabinets to disinfect the interior of the cabinet entirely. This long period of time is not so objectionable when simple operations, i.e. operations which usually are trouble free, have to be carried out in the cabinet. However, this is quite different when more complicated operations, in which, for example, more complicated and hence also more trouble sensitive devices are used, have to be carried out in the isolator. In the case of troubles of the devices in the isolator, for example, for replacing component parts or carrying our repairs, in which wearing gloves is annoying, the isolator has often to be opened, after which it will take another approximately 15 hours before the operations in the cabinet may be continued. Of course, this also applies to the necessary disinfection after opening machines or parts thereof for performing repairs, in which the interior of the isolator is exposed to non-sterilised machine parts. Such a long-lasting disinfection procedure is particularly annoying and delaying for the process which is carried out in the isolator.

The British Patent Specification 2,223,678 describes the automated supply and control of a sterilising vapour to an isolator. In the device described the sterilising vapour is introduced into the isolator via a perforated inlet plate, is passed through the isolator and leaves the isolator via two exhaust collectors. The sterilising aerial mixture is produced by means of an evaporation chamber which communicates, via a pump, with a reservoir comprising sterilising agent. The sterilising vapour leaves the isolator is via an exhaust filter and finally reaches the open air. As appears from the Figures accompanying the said Patent Specification, complete strerilisation is reached in approximately 2 hours (introducing sterilising vapour, sterilisation and rinsing together), a considerable improvement compared with the period of time necessary in the isolators now available.

However, this improvement is still insufficient. Interruptions of the process carried out in the isolator also lead to unacceptable delays in the system described in the said British Patent Specification.

It is the object of the present invention to provide a method of disinfecting the interior of an isolator, as described in the opening paragraph, which is considerably faster in reaching the desired result.

This object can be achieved by performing the method in such a manner that, according to the present invention, a liquid disinfectant is nebulised in an air flow and the said disinfecting aerial mixture is then caused to circulate laminarly through the isolator via a highly efficient particle filter (HEPA filter).

As will become apparent from the examples, it has proved possible to reduce the time necessary to disinfect the interior of the isolator and then to rinse it to well within thirty minutes, even to approximately 15 minutes; hence a very considerable time saving.

The isolator has been described hereinbefore. Of course, the interior of the isolator is not restricted to the inner walls of the isolator but also includes anything which is present inside the isolator, so materials, instruments and other devices, containers and other dispensing and packaging materials, machines, for example, dispensing machines, and the like.

Suitable disinfectants which may be used in the method according to the invention are compositions of hydrogen peroxide, of formaldehyde, of glutaraldehyde and of peroxycarboxylic acids, for example, performic acid, peracetic acid or halogenated peracetic acids, peroxypropionic acid or halogenated peroxypropionic acids, and the like.

In order to ensure that the air which is introduced into the isolator is not infected, the air flow is passed through a HEPA filter. A suitable HEPA filter comprises a glass fibre material as a filter medium, accommodated in a filter housing. It is ensured by a slight excess pressure in the isolator, for example, of at least 15 Pa, that micro-organisms cannot penetrate into the cabinet via a possible leak.

It is essential for the liquid disinfectant to be nebulised in the air flow, after which the disinfecting aerial mixture thus obtained is then circulated laminarly through the isolating cabinet via the above-described filter. Nebulising the disinfectant can best be done by providing a suitable nozzle in the air flow and forcing the disinfectant through the said nozzle by means of a dosing pump or by means of gas pressure. An essentially raised temperature is not necessary for nebulising, as a result of which the half-life of peracetic acid is not detrimentally influenced.

The circulation of the disinfecting aerial mixture enables a better control of the relative humidity and of the temperature and moreover restricts the emission.

In order to be able to disinfect the interior of the isolator as rapidly as possible, it is advantageous to ensure that the disinfecting aerial mixture is passed laminarly over the whole cross-section through the isolator, the air present being removed uniformly. In this manner the interior of the isolator, i.e. the inner walls including any devices, instruments, materials, and so on, is contacted approximately uniformly with the disinfectant and is disinfected.

A composition which comprises a peroxycarboxylic acid, preferably peracetic acid, is preferably used as a disinfectant. It has been found that such preferred compositions combine an excellent disinfecting activity and a low toxicity with a good removability by rinsing with sterile air. Peracetic acid has proved to be best suitable for this purpose. After use it is easily converted into acetic acid and hydrogen peroxide, the latter in its turn into oxygen and water; by rinsing with sterile air, all these chemical products can rapidly and easily be removed by said air. Various peracetic acid compositions which are suitable for use in the method according to the invention are commercially available. In most of the cases these compositions are stabilised by means of a suitable stabiliser, for example, hydrogen peroxide or sulphuric acid. In addition to peracetic acid the said compositions may also comprise other peroxy compounds, for example, hydrogen peroxide, in varying concentrations. The peracetic acid concentration in these compositions may vary within wide limits and is preferably as high as possible, in so far as permitted by the stability of the composition.

The present invention also relates to a device for performing the method as defined hereinefore, which device comprises a hermetically sealed isolator which is connected to an air circulation system. According to the invention the device is characterised in that at its upper side the isolator is connected, via a pressure distribution system and an air filtering system, to an air supply line and is connected at its lower side to an air outlet line, and in that a nebuliser for nebulising a liquid disinfectant is provided in the air supply line.

A so-called HEPA filter, i.e. a highly efficient particle air filter, which ensures that all particles and micro-organisms are filtered from the air, is preferably used as a filter. The air is circulated by pumping, in which a certain quantity of air is exhausted from the circulation system and is replenished by fresh open air (via a prefilter). During use of the isolator, the circulating air would be warmed up by heat-producing devices present in the isolator and by the circulation pump. The temperature of the circulating air can be controlled by admitting cool fresh air, to be controlled via a valve. It is ensured that a small excess pressure, for example, of at least 15 Pa, is present in the isolator; this is controlled by means of valves. The liquid disinfectant is automatically forced towards the nebuliser or nozzle which is accommodated in the air supply line, and is then nebulised in the air flow. The ratio between the transversal surface of the air supply line and the inner surface of the isolator is preferably approximately 1 : 10.

The comparatively wide (large diameter) inlet and outlet lines enable a rapid circulation by pumping of the sterilising aerial mixture. Since the sterilising aerial mixture is circulated by pumping, substantially no sterilising agent can be lost irrespective of the short circulation time.

As already mentioned hereinbefore it is advantageous for the disinfecting aerial mixture to be passed over the whole cross-section through the isolator as uniformly as possible. In order to realise this the pressure distribution system preferably comprises a straining cloth which extends over the whole cross-section of the isolator, while between said cloth and the filtering system, which filtering system may comprise one or more filtering units, preferably sufficient space is present to distribute the supplied air as uniformly as possible over the surface of the straining cloth. Cross-section of the isolator is to be understood to mean herein the cross-section perpendicular to the direction of the air flow. In order to achieve a distribution of the supplied air which is as uniform as possible, a so-called pressure chamber or plenum is preferably used which is situated between the filtering system and the straining cloth and which comprises a safety against excess pressure. So the object of the pressure distribution system is to allow the disinfecting aerial mixture to pass laminarly through the isolator. Filtering system and pressure distribution system are collectively referred to as laminar air flow unit.

In order to maintain the air flow laminar as long as possible during its travel through the isolator and to prevent premature deflection, the air is preferably exhausted from the isolator via a perforated bottom plate and a second plenum.

By using the above-mentioned pressure distribution system and the perforated bottom plate, any turbulence which would cause mixing of the fresh sterilising air mixture with the air present is avoided.

It may be necessary to bring materials and objects into the isolator, for example, to allow trouble shooting of the devices present therein. As already explained hereinbefore, the rapid sterilisation of the isolator which can be achieved by means of the device according to the invention is of great advantage. The isolators now available usually comprise one or more transfer airlock systems through which small objects can be introduced into the isolator in a sterile manner. The said transfer airlock systems are excellent means but they are less suitable for introducing larger objects into the isolator, for example, dispensing machines and dispensing devices, instruments, and the like. Furthermore, an isolator which must be suitable for production, dispensing, etc., usually comprises one or more leadthroughs through the wall, which leadthroughs are circumferentially hermetically sealed and are used for current supply wires and inlet and outlet lines for substances, in particular chemicals and the like. In order to enable the introduction of larger objects, a preferred embodiment of the isolator according to the invention comprises at least one inlet gate which can be sealed hermetically by means of a movable wall panel. Such a wall panel may be hingeably connected to the isolator but in the case of large inlet gates, for example, of at least approximately 1 $m^2$, it is preferably accommodated so as to be movable on wheels. After having been moved in front of the inlet gate, the wall panel is secured hermetically to the inlet gate.

According to another aspect of the present invention it has been found that an excellent hermetic seal between the wall panel and the inlet gate can be obtained by providing the isolator externally with a number of locks which can lock the movable wall panel in a sealing position with respect to the inlet gate, and by providing either the outer wall of the isolator around the inlet gate or the inside of the wall panel with a hermetic sealing system between inlet gate and wall panel, which system consists of two circumferential hollow profile gaskets which are provided around the inlet gate or on the wall panel so as to be in a mutually parallel position and at a short distance from each other and thus enclose a closed, circumferential space which is bounded by the gaskets and which has a channel-like cross-section, the hollow profile of the gaskets being connected to a compressed-air line and the space being connected to a vacuum line. The above sealing system has been found to be particularly suitable because a reliable hermetic seal is obtained also when the side walls of the isolator around the inlet gate externally or the inside of the wall panel are not absolutely flat, which is hardly avoidable in the case of large surfaces and when using comparatively thin material (plating). Such a sealing system is known for a vacuum chamber, for example, from Swedish Patent Specification 457748. It has been found surprisingly that such a sealing system may also be used excellently in a device according to the present invention in which there is no under-pressure of nearly 98 kPa which in fact would promote the sealing by suction of the sealing element, but just an overpressure, for example, of approximately 20 Pa.

The invention will now be described in greater detail with reference to the accompanying drawings, in which:

Figure 1 shows diagrammatically a suitable embodiment of the device according to the invention;

Figure 1-A is a detail sectional view of the part of the device which is referred to by A in Figure 1;

Figure 1-B is also a detail sectional view, this time of the part of the device which is referred to by B in Figure 1;

Figure 2 is a cross-sectional view of an essential detail of the sealing system between inlet gate and wall panel of the isolator shown in Figure 1; and

Figure 3 is a graph showing the sterilisation and rinsing of the device shown in Figure 1.

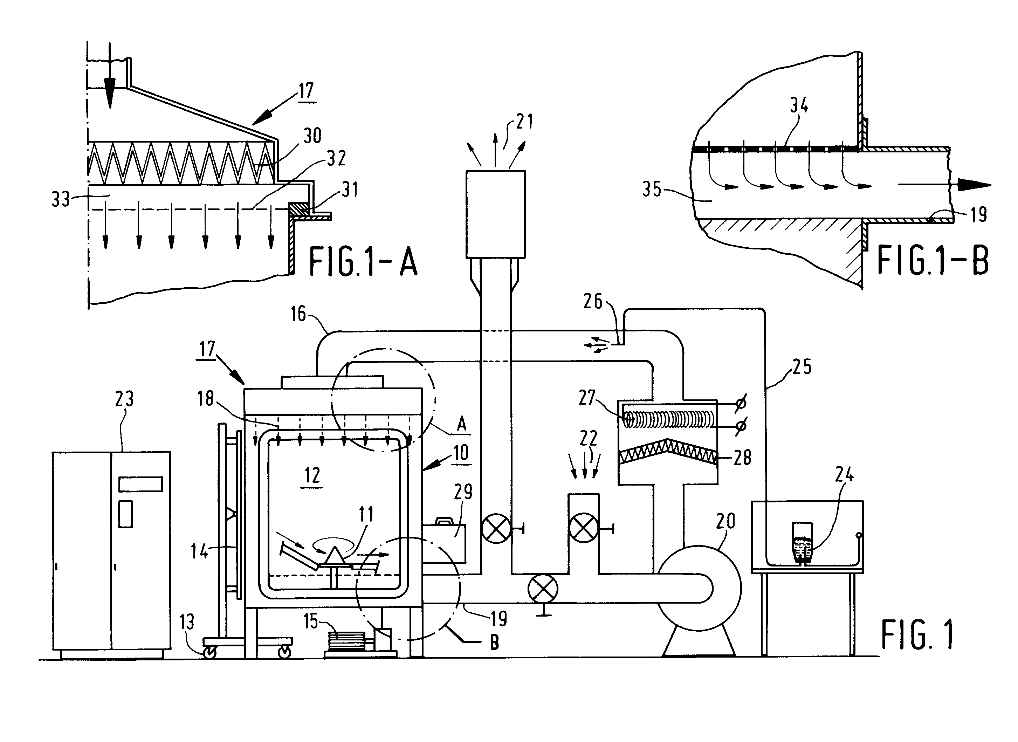

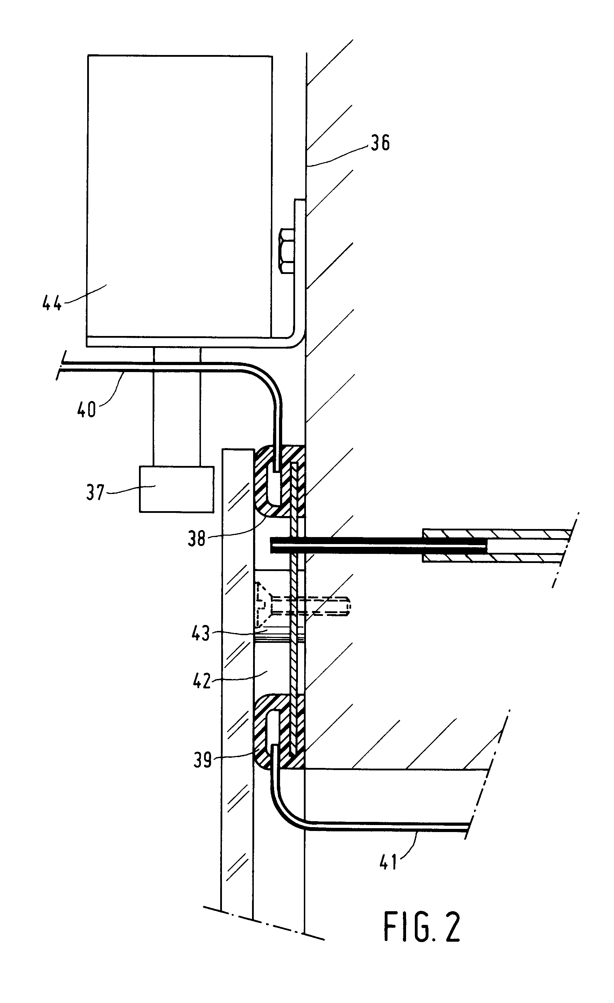

The device shown in Figure 1 comprises an isolator 10 in which a dispensing machine 11 is accommodated. The isolator cabinet is accessible at its front via an inlet gate 12 which can be sealingly closed by means of a movable wall panel which is not shown in the drawing, and at its side also via an inlet gate, equally sealed by means of a wheeled (13) wall panel 14. The sealing between the inlet gate and the wall panel will be described in more detail with reference to Figure 2. Reference numeral 15 denotes a vacuum pump. The inlet line 16 having a comparatively large transversal area, namely approximately 10% with respect to the inner bottom surface of the isolator, is connected to the top of the isolator via a so-called laminar air flow (LAF) unit 17 which ensures a laminar air flow in the isolator of approximately 0.5 m/sec.; this is denoted by arrows 18. The operation of the LAF unit will be described in greater detail with reference to Figure 1-A. The air outlet line 19 receives the air exhausted from the isolator and circulates it by means of a circulation pump 20 which is capable of circulating the air in approximately 4 seconds; a flow rate of approximately 10 m/sec is then measured in the wide inlet and outlet lines. Valves are present, inter alia to control the excess pressure and the temperature (by admitting cool fresh air) in the isolator. The deficiency resulting from blowing down (at 21) is replenished by fresh air at 22. By controlling the valves a slight excess pressure, for example, of approximately 20 Pa, can be realised in the air circulation system and hence in the isolator. The control of the whole system is accommodated in the control cabinet 23. The liquid disinfectant in reservoir 24 is forced to the nozzle 26 via supply line 25 by means of compressed air and nebulised there in the air flow. A stabilised peracetic acid solution may successfully be used as a disinfectant. During disinfecting the evaporation of the disinfectant may be stimulated by heating the circulating air flow by means of a heating element 27. A prefilter 28 ensures that particles in the admitted fresh air are filtered off. Smaller objects can be introduced into the isolator in a sterile manner via a transfer airlock unit 29.

Figure 1-A shows a part of the LAF unit 17 which ensures a laminar air flow in the isolator. For that purpose the LAF unit comprises a filtering system 30 which consists of one or more filtering units, and a straining cloth 32 tensioned in a frame 31 between which filtering system and straining cloth a pressure chamber or plenum 33 is situated. The detail sectional view of Figure 1-B shows the perforated bottom plate 34 and the second plenum 35, by which premature deflection of the air flow is prevented.

The inlet gate can be sealed hermetically by means of a movable wall panel 14 as shown in detail in Figure 2. The outside of the isolator wall 36 comprises around the inlet gate a connection means for locks 37 for locking the panel to the inlet gate; these locks are operated pneumatically (44). The hermetic sealing system between the inlet gate and panel comprises two circumferential hollow profile gaskets 38 and 39, to which a compressed-air line is connected. Tubes 40 and 41 connect the compressed-air line with the interior of the gaskets. The gaskets which are provided around the inlet gate, which extend mutually parallel and which are manufactured from an elastic material, preferably a silicone rubber, enclose a closed circumferential space 42 having a channel-shaped cross-section which is connected to the vacuum pump 15. Spacers 43 connected to the wall constitute an abutment for the wall panel. The sealing of the inlet gate by the wall panel proceeds as follows. The wall panel is situated in the correct position for closing and is locked. The vacuum pump is then actuated after which the hollow gaskets are pumped until a hermetic seal is obtained. Only then is the space 42 evacuated and may pumping be discontinued.

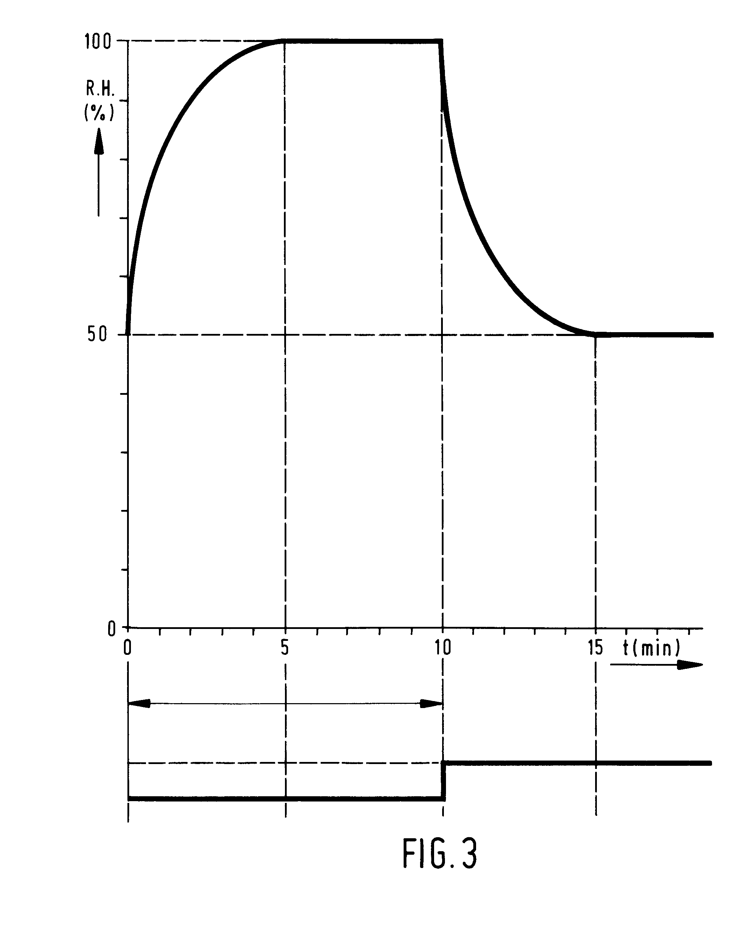

That the use of the device shown in Figure 1 very rapidly leads to the desired result may be illustrated with reference to the curve shown in Figure 3. In this graph the relative humidity is plotted against time. The curve shown is obtained by measuring the relative humidity in the isolator during the disinfecting process: nebulising and evaporating the disinfecting solution (t = 0), disinfecting the interior of the isolator after discontinuing the nebulising (t = 5 min.), and rinsing with sterile air (t = 10 min.). After 15 minutes the relative humidity has returned to the original level and the isolator may be used. It has been found that the number of micro-organisms in the isolator at that instant has reduced sufficiently for performing operations in the isolator under aseptic conditions. The variation of the relative humidity is measured in the control cabinet.

## Claims

1. A method of disinfecting the interior of an isolator comprising a laminar air flow system, characterised in that a liquid disinfectant is nebulised in an air flow and that the said disinfecting aerial mixture is then caused to circulate laminarly through the isolator via a highly efficient particle filter.

2. A method as claimed in Claim 1, characterised in that the disinfecting aerial mixture is caused to circulate laminarly through the isolator over its whole cross-section.

3. A method as claimed in Claim 1 or 2, characterised in that a composition which comprises a peroxycarboxylic acid, preferably peracetic acid, is used as a disinfectant.

4. A device for performing the method as claimed in any of the preceding Claims comprising a hermetically sealed isolator which is connected to an air circulation system, characterised in that at its upper side the isolator is connected to an air supply line via a pressure distribution system and an air filtering system, and at its lower side the isolator is connected to an air outlet line, and in that a nebuliser for nebulising a liquid disinfectant is provided in the air supply line.

5. A device as claimed in Claim 4, characterised in that the pressure distribution system comprises a straining cloth which extends over the whole cross-section of the isolator and in that a pressure chamber (plenum) is situated between the air filtering system and the straining cloth.

6. A device as claimed in Claim 4 or 5, characterised in that the isolator is connected to the air outlet line via a perforated bottom plate and a pressure chamber (plenum).

7. A device as claimed in any of the Claims 4-6, characterised in that the isolator which is optionally provided with one or more circumferentially hermetically sealed leadthroughs through the wall, comprises at least one inlet gate which can be sealed hermetically by means of a movable wall panel.

8. A device as claimed in Claim 7, characterised in that the isolator externally comprises a number of locks capable of locking the movable wall panel in a sealing position with respect to the inlet gate, and in that either the outer wall of the isolator around the inlet gate or the inside of the movable wall panel comprises a hermetic sealing system between inlet gate and wall panel, comprising two circumferential hollow profile gaskets which are provided in a mutually parallel position and at a short distance from each other around the inlet gate or on the wall panel and thus enclose a closed, circumferential space which is bounded by the gaskets and which has a channel-like cross-section, the hollow profile of the gaskets being connected to a compressed-air line and the space being connected to a vacuum line.

FIG.1-A

FIG.1-B

FIG.1

EP 0 475 505 A1

FIG. 2

FIG.3

European
Patent Office

EUROPEAN SEARCH
REPORT

Application Number

EP 91 20 2231

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | GB-A-2 223 678 (CAMBRIDGE ISOLATION TECHNOLOGY LTD) <br> * page 2, paragraph 2 - page 3, paragraph 2; claims; figures * * | 1-8 | A 61 L 2/22 <br> A 61 L 9/14 |
| Y | US-A-4 098 174 (J.LANDY) <br> * claims; figures * * | 1-8 | |
| A | FR-A-2 255 082 (DRAGERWERK AG) <br> * claims * * | 1-8 | |
| A | DE-A-1 903 137 (GIO &F.LLI BUITONI SANSEPOLCRO PERUGIA) <br> * claims 1-3 * * | 1-8 | |
| A | FR-A-2 605 246 (EQUIPEMENTS SCIENTIFIQUES & INDUSTRIELS FLUFRANCE) | | |

|  | TECHNICAL FIELDS SEARCHED (Int. Cl.5) |
|---|---|
|  | A 61 L <br> B 01 L <br> F 24 F |

The present search report has been drawn up for all claims

| Place of search | Date of completion of search | Examiner |
|---|---|---|
| The Hague | 12 November 91 | COUSINS-VAN STEEN G. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document